# EUROPEAN PATENT SPECIFICATION

(11) **EP 0 618 807 B1**
(45) Date of publication and mention of the grant of the patent: **13.11.2002**
(21) Application number: 93902089.7
(22) Date of filing: 16.12.1992
(51) Int. Cl.: A61K 38/27

(54) **A STABILIZED PHARMACEUTICAL FORMULATION COMPRISING GROWTH HORMONE AND HISTIDINE**
STABILISIERTE PHARMAZEUTISCHE FORMULIERUNG, DIE WACHSTUMSHORMON UND HISTIDIN ENTHÄLT
FORMULATION PHARMACEUTIQUE STABILISEE COMPORTANT UNE SOMATOTROPHINE ET DE L'HISTIDINE

(30) Priority: 20.12.1991 DK 204691; 10.11.1992 DK 136492
(43) Date of publication of application: 12.10.1994
(60) Divisional application: 01128974.1
(73) Proprietor: NOVO NORDISK A/S, 2880 Bagsvaerd (DK)
(72) Inventor: SORENSEN, Hans, Holmegaard, DK-2830 Virum (DK); SKRIVER, Lars, DK-2930 Vedbaek (DK); HOELGAARD, Annie, Rassing, DK-2840 Holte (DK)
(74) Representative: Nilausen, Kim
(86) International application number: DK9200379
(87) International publication number: WO93012812

(56) References cited:
- EP-A- 0 374 120
- US-A- 4 816 568
- US-A- 4 917 685

## Description

### FIELD OF THE INVENTION

The present invention relates to a stabilized pharmaceutical formulation comprising human growth hormone and histidine or a derivative of histidine, to a method of making such formulation, crystals of human growth hormone comprising histidine or a derivative thereof, a method of preparing such crystals and the use of histidine or derivatives of histidine for stabilizing a formulation of human growth hormone.

### BACKGROUND OF THE INVENTION

The growth hormones (GH) from man and from the common domestic animals are proteins of approximately 191 amino acids, synthesized and secreted from the anterior lobe of the pituitary gland. Human growth hormone consists of 191 amino acids.

Growth hormone is a key hormone involved not only in the regulation of somatic growth, but also in the regulation of metabolism of proteins, carbohydrates and lipids. The major effect of growth hormone is to promote growth.

The organ systems affected by growth hormone include the skeleton, connective tissue, muscles, and viscera such as liver, intestine, and kidneys.

Until the development of the recombinant technology and the cloning of the growth hormone gene now giving rise to production of e.g. human growth hormone (hGH) and Met-hGH on an industrial scale, human growth hormone could only be obtained by extraction from the pituitary glands of human cadavers. The very limited supplies of growth hormone restricted the use thereof to longitudinal growth promotion in childhood and puberty for treatment of dwarfism, even though it has been proposed for inter alia treatment of short stature (due to growth hormone deficiency, normal short stature and Turner syndrom), growth hormone deficiency in adults, infertility, treatment of burns, wound healing, dystrophy, bone knitting, osteoporosis, diffuse gastric bleeding, and pseudoarthrosis.

Furthermore, growth hormone has been proposed for increasing the rate of growth of domestic animals or for decreasing the proportion of fat in animals to be slaughtered for human consumption.

Pharmaceutical preparations of growth hormone tend to be unstable. Degradation products such as deamidated or sulfoxydated products and dimer or polymer forms are generated - especially in solutions of growth hormone.

The predominant degradation reactions of hGH are 1) deamidation by direct hydrolysis or via a cyclic succinimide intermediate to form various amounts of L-asp-hGH, L-iso-asp-hGH, D-asp-hGH, and D-iso-asp-hGH (ref 1-3), 2) oxidation of the methionine residues in positions 14 and 125 (ref 4-9), and 3) cleavage of peptide bonds.

Deamidation especially takes place at the Asn in position 149.

hGH is rather easily oxidized in positions 14 and 125, especially in solution (4-8).

The oxidation of hGH in solution forming sulfoxides is normally due to the oxygen dissolved in the preparation. The solubility of oxygen in distilled water is about 200 µM (9). As the concentration of hGH in a preparation comprising 4 IU/ml is 1.3 mg/ml corresponding to 60 nM hGH, oxygen will, under normal storage conditions, be present in an excess of about 3000 times the stoichiometric amount for oxidation of hGH. It is not feasible to try to solve the problem by degassing of buffers before tapping and packing the preparations.

At present, it is not believed that these degradation products should have toxic or altered biological activity or receptor binding properties, but there is indication to the effect that the conformation stability of the sulfoxides is reduced as compared to native hGH.

For the development of a stable, dissolved preparation comprising hGH it is of importance to know the rate of formation of sulfoxides as well as means to control the oxidation.

The kinetics of degradation depend on temperature, pH and various additives or adjuvants in the hGH formulation.

Due to the instability, growth hormone is, at present, lyophilized and stored in the lyophilized form at 4°C until it is reconstituted for use in order to minimize the degradation.

The lyophilized pharmaceutical preparations comprising hGH are, at present, reconstituted by the patient and then stored at a low temperature, often at about 4°C in the refrigerator as a solution during the period of use of up to 14 days, during which some degradation will take place.

Furthermore, the process of reconstitution of the lyophilized growth hormone tends to provide difficulties for the patient.

Thus, it is at present preferred to reconstitute the growth hormone as late as possible before use and to store and ship the preparation in a lyophilized state. The chain from the manufactorer to the pharmacy is apt for handling the preparations at a controlled low temperature of e.g. 4°C which allows for a long shelf life of up to two years.

However, the extended use of pen systems for self-medication and the expanded field of use calls for a preparation which is stable for a sufficiently long time with the end user under conditions where "sufficient" cooling is not always available.

Preferably, a preparation should be stable with the end user in a lyophilized state for about one month and additionally for one month in a reconstituted state in a pen device for the intended period of use of a cartridge.

Thus, there is a need for more stable preparations of growth hormone being stable in a lyophilized state at a relatively high temperature for a period and additionally for a period of use at a relatively high temperature in solution. Such stabilization is of very great importance when moving the administration of the growth hormone from clinics to the homes of the individuals to be treated where optimal storage may not be available as indicated above.

Furthermore, the shift in pattern of administration of growth hormone to the use of pen devices calls for a stable dissolved preparation comprising growth hormone in order to facilitate the handling to be performed by the patient. A stable dissolved preparation comprising growth hormone may be produced ready to use in the form of cartridges fitting into the pen device used by the patient who may then avoid the reconstitution of the preparation and, hence, will not have to be in the possession of a lyophilized preparation, a suitable vehicle for reconstitution as well as the necessary skill and sterile equipment for sterile reconstitution of the preparation.

For safety reasons it will also be desirable to avoid the reconstitution of a lyophilized preparation just before the use of the preparation.

Furthermore, it would also be an advantage to avoid the lyophilization step in the production of growth hormone preparations. Lyophilization is a time consuming and costly process and is also often a "bottleneck" in the production due to the limited capacity of the freeze drier.

Thus, there is a need to reduce the rate of the degradation processes in order to allow for dissolved hGH preparations being stable during shelf life and during the period of use of up to one month.

Prior attempts to stabilize hGH have not fully succeeded in preventing the formation of dimer. The problems associated with dimer formation are, e.g., noted in Becker, G.W., Biotechnology and Applied Biochemistry 9, 478 (1987).

International Patent Publication No. WO 89/09614 and Australian patent application No. 30771/89 disclose a stable pharmaceutical formulation containing human growth hormone, glycine, and mannitol. Such a preparation shows improved stability during normal processing and storage in a lyophilized state as well as in the period of use after the reconstitution.

Published European patent application No. 303 746 discloses that animal growth hormone may be stabilized with various stabilizers to give decreased formation of insolubles and preservation of the soluble activity in aqueous environments, such stabilizers including certain polyols, amino acids, polymers of amino acids having a charged side group at physiological pH, and choline salts. Polyols are selected from the group consisting of nonreducing sugars, sugar alcohols, sugar acids, pentaerythritol, lactose, water-soluble dextrans and Ficoll; amino acids are selected from the group consisting of glycine, sarcosine, lysine and salts thereof, serine, arginine and salts thereof, betaine, N,N,-dimethyl-glycine, aspartic acid and salts thereof, glutamic acid and salts thereof; a polymer of an amino acid having a charged side group at physiological pH may be selected from polylysine, polyaspartic acid, polyglutamic acid, polyarginine, polyhistidine, polyornithine and salts thereof; and choline derivatives are selected from the group consisting of choline chloride, choline dihydrogen citrate, choline bitartrate, choline bicarbonate, tricholine citrate, choline ascorbate, choline borate, choline gluconate, choline phosphate, di(choline)sulphate and dicholine mucate.

EP 374120 discloses a stabilized preparation of growth hormone comprising a buffered polyol excipient comprising a polyol having three hydroxy groups and a buffer to achieve a pH in a range in which the growth hormone retains its bioactivity for a sufficient period of time. Histidine is mentioned as a buffer for a polyol having three hydroxy groups. In the last mentioned EP application, none of the specific examples relate to human growth hormone. Furthermore, in this application there is no mention of deamidation of human growth hormone.

### BRIEF DESCRIPTION OF THE INVENTION

It has now surprisingly been found that a preparation of human growth hormone comprising only histidine or a derivative thereof as additive or buffering substance in an amount of from 0.1 to 12 mg histidine or derivative thereof per mg of growth hormone shows a very high stability against deamidation, oxidation and cleavage of peptide bonds. The stability of the product allows for the storage and shipment thereof in a lyophilized state or in the form of a dissolved or re-dissolved preparation.

EP 303746 mentions polyhistidine as a potential stabilizer for animal growth hormone, but there is no indication whether it stabilizes an animal growth hormone or human growth hormone. In the last mentioned EP application, none of the specific examples relate to human growth hormone. Furthermore, in this application there is no mention of deamidation of human growth hormone.

EP 374120 teaches that histidine hydrochloride may be used as a buffer for buffering a polyol having three hydroxy groups for improving the stability of a growth hormone preparation in the form of a solution comprising a high concentration of growth hormone and a polyol as stabilizer. Histidine hydrochloride must be added in an amount of about 3% by weight of the solution corresponding to a concentration of -0.15 M solution of histidine hydrochloride. EP 374120 also teaches that histidine alone does not impart chemical and physical stability to a growth hormone preparation.

The preparation of the invention may be in the form of a lyophilized powder to be reconstituted later using conventional vehicles such as distilled water or water for injection or in the form of a solution or a suspension of crystals comprising growth hormone. Such vehicles may comprise conventional preservatives such as m-cresol or benzyl alcohol.

A preferred embodiment of the invention is in the form of a pharmaceutical preparation of human growth hormone comprising histidine or a derivative thereof in the form of a buffered aqueous solution of growth hormone buffered with histidine buffer. Such a preparation is in a ready-to-use form and may be stored and shipped as an aqueous solution without any significant degradation. L-histidine has a pKA of 6.0 and is, accordingly, suitable as a buffer itself at pH 6.5.

The formulation with histidine at pH 6.5 is considered stable at 25°C for almost 50 days.

A further, preferred embodiment of the invention is in the form of a pharmaceutical preparation of human growth hormone comprising histidine or a derivative thereof in the form of a buffered aqueous suspension of crystals of growth hormone buffered with histidine buffer. Such a preparation is very stable and keeps the growth hormone in a crystalline phase during the storage and shipment in a ready-to-use form giving an even lower tendency to degrade. Such a preparation acts like a dissolved preparation when injected, i.e. there is no sustained release of the human growth hormone.

For stability reasons the pH of a solution or suspension preparation is preferably adjusted to a value in the interval from 2-9. Preparations having a pH from 5 to 8 and especially a pH from 6 to 7.5 are more preferred.

In order to obtain the stabilizing effect, the concentration of histidine is preferably from 1 mM to 100 mM. More preferably, the concentration of histidine added is in an amount from 2 to 20 mM, most preferably from 5 to 15 mM.

Addition of 10% ethanol or 5% methanol resulted in more than 20% reduction in the deamidation.

The preparations of the invention may also be in the form of a lyophilized powder or "cake" comprising human growth hormone and histidine or a derivative thereof in an amount from 0.1 to 12 mg histidine or derivative thereof per mg growth hormone, and a bulking agent for lyophilization selected from the group consisting of sugar alcohols and disaccharides and mixtures thereof. A sugar alcohol is preferably mannitol.

Lyophilized preparations according to the invention comprising sucrose are preferred due to a very high stability, and preparations comprising sucrose and mannitol are especially preferred, combining very high stability with very good processability giving firm lyophilized products being readily dissolvable and very stable in solution for an extended period of time after dissolution. Further preferred preparations according to the invention are preparations comprising mannitol and trehalose as bulking agent for the lyophilization. Preparations according to the invention comprising mannitol and a disaccharide normally comprise about equal amounts of the two constituents on a weight basis.

The amount of sucrose present in the preparations of the invention may vary within wide limits. The ratio of growth hormone to sucrose may vary from 0.005 to 1.5 on a weight basis. Thus, the amount of sucrose may be from 0.67 to 200 mg per mg of growth hormone, an amount of from 1.1 to 50 mg per mg of growth hormone being preferred.

Lyophilization of hGH in histidine buffer does not give rise to any problems. The rate of deamidation is reduced by 20% on standing after redissolving, as compared to phosphate buffer.

The pharmaceutical preparation of the invention may furthermore comprise salts conventionally used in order to facilitate the processing thereof, e.g. the lyophilization or reconstitution.

Another way of stabilizing human growth hormone according to the invention is to form crystals of human growth hormone giving a good protection against degradation. It has surprisingly been found that preparations of human growth hormone in the form of crystals comprising histidine fulfil the above-mentioned needs. The crystals in dried form may be used directly as a GH preparation to be reconstituted before use in the conventional manner.

Thus, the present invention also relates to crystals of human growth hormone comprising histidine or a derivative thereof and an organic or inorganic cation. It has been shown that the quality of such crystals is better than the quality obtained using previous formulations.

Although readily available in quantities sufficient for crystallization, no successfull crystallization of GH has been reported so far. Micro crystals, or amorphous material, have been reported from a variety of sources: Jones et al., BioTechnology (1987) 5, 499 - 500; Wilhelmi et al., J.Biol.Chem. (1984) 176, 735 - 745; Clarkson et al., J.Mol.Biol. (1989) 208, 719 - 721; and Bell et al., J.Biol.Chem. (1985) 260, 8520 - 8525.

The hanging drop method is the most common method used in attempts for crystallizing GH. Apparently due to heterogenicity growth hormone preparations the size and the shape of the crystals reported vary significantly. The largest crystals have been reported by Jones et al. (1987). For their successfull experiments they used a mixture of polyethylene glycol 3500 and beta octyl glucoside at neutral pH. Clarkson et al. (1989) reported that the use of lower alcohols and acetone permitted the generation of crystals of 0.001 to 0.005 cubic mm with varying shapes. None of the known methods are, however, suitable for commercial production of GH crystals, a.o. due to the fact that periods of growth of from several weeks up to one year are needed.

Bovine growth hormone has been formulated for veterinary use in a mixture of divalent ions and an oil (EP 343 696). By addition of ZnCl₂ to either bovine or porcine growth hormone in the presence of lipids, undefined particles were produced to form a prolonged release formulation. The growth hormone was dispersed in the carrier in such a way as to trap 1 to 4 Zn molecules per growth hormone molecule. The solutions were prepared in the presence of varying concentrations of denaturing solutes (1 to 4 M of urea) at high pH (9.5). A reproduction of this process with hGH has shown that it is not possible to produce crystals in this way.

From the literature it is well known that the presence of divalent cations during the process of crystallization of insulin permits not only excellent orientation during analysis, but also improved physical conditions for the crystallization (see e.g. US pat. no. 2174862). Growth hormone is, however, more than three times larger than insulin and has a totally different conformation. Surprisingly, it has now been shown that the addition of cations to solutions containing hGH and histidine or a derivative of histidine renders possible the generation of stable, uniform crystals of the growth hormone in high yields. Furthermore, the period of time necessary for the formation of high quality crystals of hGH is relatively short.

A further aspect of the invention is a method for preparing crystals of human growth hormone and histidine or a derivative of histidine comprising the steps of:
a) forming a solution of human growth hormone in a solvent and adding histidine or a derivative of histidine and optionally adjusting the pH to a value from 5 to 8 using hydrochloric acid,
b) adding organic or inorganic cations,
c) crystallizing the solution at a temperature from about 0°C to about 30°C, and
d) isolating the crystals formed in a manner known per se.

It has been found that crystallizing hGH in the presence of histidine or a derivative thereof gives a higher yield of crystalline hGH in the form of larger and more pure and uniform crystals than crystallization in the presence of phosphate buffer normally used for formulation of preparations of hGH. Thus, the isolation and purification of the crystals is facilitated.

The yield of crystals has been increased by - 20% when carrying out the crystallization in the presence of histidine, as compared with crystallization from previous formulations.

The starting material, the human growth hormone, may be a concentrate obtained directly from the fermentation broth or a conventional lyophilized preparation which is dissolved in the solvent and adjusted to a concentration of preferably more than 0.1 mg/ml, more preferably a concentration from 4 to 7 mg/ml and most preferably a concentration of 6 mg/ml. The solvent used in step a) is suitably an aqueous buffer such as phosphate buffer or histidine buffer.

The crystallization is allowed to proceed for a period of from 1 to 120 hours, preferably from 5 to 72 hours and most preferably from 20 to 48 hours at a temperature. The temperature is preferably from 4 to 25°C.

The pH in step a) is normally from 5.0 to 7.5, preferably from 5.0 to 6.8, more preferably from 5.8 to 6.5, and most preferably from 6.0 to 6.3.

The concentration of histidine or histidine derivative in step a) may vary from 5-25 mM, 5-15 mM being preferred, in order to have crystals of appropriate size and quality as stated above.

Divalent cations are preferred, and inorganic cations such as Zn⁺⁺ has been found to be well suited for the fast formation of stable GH crystals. Also mixtures of cations can be used.

The cation should be added in an amount providing fast and efficient formation of well defined crystals. The upper limit for the amount of added cation is the amount which would cause unspecific precipitation of substantial amounts of amorphous material.

When using Zn⁺⁺, suitable concentrations will typically be from about 0.2 to 10 mol Zn⁺⁺/mol GH. However, if the crystallization reaction mixture contains a buffer or other compound which is capable of binding the cation, e.g. in a complexed form, a higher added concentration of the cation will be needed for the crystallization process in order to compensate for this binding.

Zn⁺⁺ will preferably be used in an amount which will cause formation of hGH crystals having a molar ratio between Zn⁺⁺ and hGH from about 0.2 to about 10, more preferably from about 0.5 to about 5, and most preferably from about 0.5 to about 2.

When using other inorganic cations, the concentration may be varied between 0.5 and 10 mol cation/mol hGH.

In a preferred embodiment of the invention an organic solvent or a mixture of organic solvents is added in step a).

Suitable organic solvents to be added for the crystallization may be chosen from short chained aliphatic, alicyclic and aromatic alcohols and ketones, such as methanol, ethanol, 1- and 2-propanol, cyclohexanol, acetone, and phenol or m-cresol. Preferred organic solvents are ethanol and acetone, ethanol being most preferred.

The solution may be seeded by adding small and well defined crystals of hGH of hexagonal or needle shape, but preferably no seeding is carried out.

The concentration of the organic solvent may be from 0.1 to 50% v/y, preferably from 0.1 to 30%, more preferably from 0.1 to 20%, even more preferably from 5 to 15%, and most preferably from 6 to 12% v/v.

The present process may be used as a fast and efficient downstream processing of the human growth hormone in question, due to the formation of crystals in large volumes of solutions.

When using ethanol as the organic solvent, the concentration is suitably between 0.1 and 20%, preferably between 5 and 15% and more preferably from 6 to 12% (v/v).

The crystals formed may be isolated by conventional methods such as centrifugation or filtration, washing and optionally lyophilization to remove traces of organic solvents.

The size of the crystals will be dependent on the Zn⁺⁺ to hGH ratio and the choice and content of solvent used in the process.

hGH crystals according to the present invention have been shown to have a biological potency similar to that of a solubilized hGH standard in in vitro tests. The novel hGH crystals can thus be used for the same indications as the commercially available hGH preparation.

Still another aspect of the invention relates to the use of histidine or a derivative thereof for the preparation of a stabilized preparation of human growth hormone.

The pharmaceutical preparations of the invention are preferably presented in a unit dosis form comprising from 4 IU to 100 IU human growth hormone per dosis.

The human growth hormone used in accordance with the invention may be native human growth hormone isolated from the natural source, e.g. by extracting pituitary glands in a conventional manner, or human growth hormone produced by recombinant techniques, e.g as described in E.B. Jensen and S. Carlsen in Biotech and Bioeng. 36, 1-11 (1990).

The term "derivatives of histidine" is used, for the present purpose, to designate amides and esters of histidine such as the methyl or ethyl ester, dipeptides such as His-Gly, His-Ala, His-Leu, His-Lys, His-Ser and His-Phe, and analogues or derivatives of His such as imidazole, des-amino-His or poly-His, histidinol, imidazole-4-acetic acid and histamine. For the sake of simplicity, the content of histidine or a derivative thereof in the preparations of the invention is calculated and using the molar weight of histidine itself.

The term "salts" used to designate additional agents for facilitating the processing or reconstitution of pharmaceutical preparations comprises conventional additives such as alkali metal, alkaline earth metal or ammonium salts of organic acids such as citric acid, tartaric acid or acetic acid, e.g. sodium citrate, sodium tartrate or sodium acetate, or of mineral acids such as hydrochloric acid, e.g. sodium chloride.

In the present context, "high stability" is obtained when the preparation is more stable than the conventional formulations comprising phosphate buffer.

A "sugar alcohol" may e.g. be mannitol, xylitol, erythritol, threitol, sorbitol or glycerol.

In the present context, "disaccharide" is used to designate naturally occurring disaccharides such as sucrose, trehalose, maltose, lactose, sepharose, turanose, laminaribiose, isomaltose, gentiobiose or melibiose.

The solvent used in the preparations of the invention may be water, alcohols such as ethyl, n-propyl, isopropyl or butyl alcohol, or a mixture thereof. The solvent may comprise a preservative such as m-cresol or benzyl alcohol.

### BRIEF DESCRIPTION OF THE DRAWINGS

The invention is described in more detail with reference to the drawings, in which
Fig. 1 shows a photograph of crystals of hGH prepared in the presence of phosphate buffer (without addition of histidine) (Magnification 400X), and
Fig. 2 shows a photograph of crystals of hGH according to the invention formed in the presence of histidine buffer. (Magnification 400X).

### DETAILED DESCRIPTION OF THE INVENTION

The invention is explained in more detail in the Examples below which illustrate the invention.

### EXPERIMENTAL PART

### EXAMPLE 1.

### Reduction of the deamidation.

The rate of deamidation was examined at 37°C for hGH preparations comprising 4IU and 12IU at pH 6.5 in His buffer as compared to phosphate buffer at the same pH.

The hGH preparation comprising 4 IU having the composition A was prepared by dissolving 13.3 mg hGH in 10 ml 10 mM histidine buffer prepared by dissolving 15.5 mg histidine in 10 ml deionized water containing 0.9% benzyl alcohol and adding 0.1 N hydrochloric acid to pH 6.5. The preparation comprising 12 IU was prepared by dissolving 40 mg hGH in the same constituents as stated above.

The hGH preparation comprising 4IU having the composition B was prepared by dissolving 13.3 mg hGH in 10 ml 10 mM disodium phosphate prepared by dissolving 17.8 mg disodium-hydrogen-phosphate in 10 ml deionized water containing 0.9% (v/v) of benzyl alcohol and adding 0.1 N phosphoric acid to pH 6.5. The preparation comprising 12 IU was prepared by dissolving 40 mg hGH in the same constituents as stated above.

### Composition A:

10 mM His
0.9% benzyl alcohol
HCl to pH 6.5

### Composition B:

10 mM disodium phosphate
0.9% benzyl alcohol
phosphoric acid to pH 6.5

The preparations were examined by IE-HPLC for their content of desamido-hGH immediately after the reconstitution and after 7 days at 37°C. The results are shown in Table 1 below.

**TABLE 1.**

| Deamidation. | | |
|---|---|---|
| | Preparation | Desamido % |
| Buffer A | 4IU/ml | 1.7 |
| Start | 12 IU/ml | 2.1 |
| | | |
| Buffer A | 4IU/ml | 10.1 |
| 7 days at 37°C | 12IU/ml | 10.4 |
| | | |
| Buffer B | 4 UI/ml | 1.8 |
| Start | 12 IU/ml | 2.3 |
| | | |
| Buffer B | 4 UI/ml | 16.9 |
| 7 days at 37°C | 12 IU/ml | 14.9 |

From the above results it appears that the deamidation of hGH is significantly reduced at 37°C in histidine buffer as compared with phosphate buffer.

### EXAMPLE 2:

### Reduction of the deamidation in the presence of histidine or histidine derivatives.

The rate of deamidation was examined at 25°C for hGH preparations comprising 6 IU hGH at pH 6.5 and at pH 7.3 in 5 mM, 10 mM and 100mM His buffer as compared to 8 mM phosphate buffer at the same pH. Furthermore, the histidine derivatives His-Gly, His-Ala, His-Leu, His-Lys, His-Phe, His-Ser, Histidine methyl ester, histidinol, imidazole, imidazole-4-acetic acid and histamine were tested.

The hGH preparations were prepared by dissolving 20 mg hGH in 10 ml of histidine buffer of the desired strength prepared by dissolving 7.8 mg, 15.5 mg and 155.2 mg, respectively, of histidine in 10 ml deionized water containing 0.9% (v/v) of benzyl alcohol and adding 0.1 N hydrochloric acid to achieve the stated pH.

The hGH formulations stated in Table 2 below were stored at 25°C and analyzed for their desamido content after 14 and 30 days by IE-HPLC. The results are shown in Table 2 below.

**Table 2.**

| Content of desamido hGH as determined by IE-HPLC as a function of the formulation and the time in solution at 25°C: | | |
|---|---|---|
| Formulation (*) | Formation of desamido compound at 25°C | |
| | 14 days (') | 30 days |
| 5 mM His pH 6.5 | 6.5 | 9.1 |
| | | |
| 5 mM His pH 7.3 | 11.0 | 17.4 |
| | | |
| 10 mM His pH 6.5 | 6.8 | 9.7 |
| | | |
| 10 mM His pH 7.3 | 11.3 | 16.6 |
| | | |
| 100 mM His pH 6.5 | 9.8 | 15.2 |
| | | |
| 100 mM His pH 7.3 | 19.3 | 28.8 |
| | | |
| 8 mM di-Na-Phosfat pH 6.5 | 7.8 | 10.8 |
| | | |
| 8 mM di-Na-Phosfat pH 7.3 | 15.2 | 20.3 |
| | | |
| 8 mM di-Na-phosfat pH 6.5, 0.3% m-cresol | 9.4 | 13.2 |
| | | |
| 10 mM Asp, pH 6.5 | 21.7 | nd |
| | | |
| 10 mM Glu, pH 6.5 | 14.8 | nd |
| | | |
| 10 mM His-Gly, pH 6.2 | 5.6 | 8.1 |
| | | |
| 10 mM His-Ala pH 6.5 | 6.2 | 8.5 |
| | | |
| 10 mM His-Leu pH 6.5 | 8.8 | 12.3 |
| | | |
| 10 mM His-Lys pH 6.5 | 8.6 | 12.0 |
| | | |
| 10 mM His-Phe pH 6.5 | 7.5 | 11.3 |
| | | |
| 10 mM His-Ser pH 6.3 | 22.0 | nd |
| | | |
| 10 mM His-methylester,pH 6.5 | 4.6 | 5.2 |
| | | |
| 10 mM histidinol pH 6.5 | 27.4 | nd |
| | | |
| 10 mM imidazole pH 6.5 | 9.2 | 12.2 |
| | | |
| 10 mM imidazole-4-acetic acid pH 6.5 | 10.3 | 14.2 |
| | | |
| 10 mM Histamine pH 6.5 | 9.8 | 12.2 |

| | | |
|---|---|---|
| *: Comprises 0.9% benzyl alcohol, except formulation No.9 The content of desamido-hGH in starting material was: 2.1 % | | |

From the above Table 2 it appears that the de-amidation of hGH is reduced by approximately 20% by the addition of histidine as compared with phosphate buffer at pH 6.5 and 7.3. Furthermore, a reduction of the pH from 7.3, being the conventional pH of commercial hGH preparations, to 6.5 in itself gives rise to a reduction of the rate of de-amidation by 50%.

Histidinol does not seem to stabilize the preparations under the test conditions, and addition of histidine in larger amounts does not increase but rather detracts from the desired effect.

Comparable results are seen to be obtained using histidine analogues such as imidazole, histamine and imidazole-4-acetic acid as well as histidine methyl ester giving rise to the formation of only 3.1% desamido-hGH after 30 days at 25°C, allowing for a life-time of the preparation of 3-4 months.

Addition of Asp or Glu increases the rate of deamidation as compared to phosphate at pH 6.5.

Addition of dipeptides of the type His-X shows a positive effect for His-Gly and His-Ala, whereas His-Ser reduces the stability to de-amidation.

The above results show that the rate of de-amidation is reduced by lowering the pH and by adding histidine in a low concentration, preferably about 5mM-10mM. The rate of de-amidation may be reduced by more than 50% by lowering the pH and replacing phosphate buffer with histidine.

The use of m-cresol or benzyl alcohol as preservative seems to have no influence on the rate of de-amidation.

Split-formation (hydrolysis of peptide bonds) is reduced by histidine at pH 6.5 in comparison with phosphate.

### EXAMPLE 3.

### Reduction of the formation of sulfoxide.

The dependency on the pH and the type of buffer was examined.

### Dependency on pH:

### Formulation:

A commercial hGH preparation (Norditropin®, 12 IU/ml) comprising bicarbonate, glycine and mannitol + 0.9% benzyl alcohol was adjusted to pH 8.3, 8.0, 7.5, 7.0, 6.5 and 6.0 using 0.1 N hydrochloric acid, and the samples were left at 37°C. Analysis was carried out by RP-HPLC after 0, 7 and 14 days. The results are shown in Table 3 below.

**Table 3.**

| Formation of Sulfoxide | | | |
|---|---|---|---|
| Sample | Temp °C | Days | Sulfoxide % |
| pH 8.37 | - | 0 | 1.0 |
| pH 8.37 | 37 | 7 | 9.0 |
| pH 8.04 | 37 | 7 | 8.7 |
| pH 7.52 | 37 | 7 | 8.3 |
| pH 7.01 | 37 | 7 | 7.7 |
| pH 6.52 | 37 | 7 | 6.5 |
| pH 6.02 | 37 | 7 | 4.8 |
| pH 8.37 | 37 | 14 | 14.9 |
| pH 8.04 | 37 | 14 | 14.5 |
| pH 7.52 | 37 | 14 | 14.0 |
| pH 7.01 | 37 | 14 | 12.9 |
| pH 6.52 | 37 | 14 | 11.1 |
| pH 6.02 | 37 | 14 | 7.7 |

As will be apparent, the formation of sulfoxide of hGH is reduced when lowering the pH from 8.4 to 6.0.

### Type of Buffer, pH:

A B-hGH preparation comprising 12 mg/ml distilled water was diluted in the proportion 1+10 with various buffers in a concentration of 15 mM and optional further added additive(s). The samples were left at 25°C, and analysis by RP-HPLC was carried out after 10 and 34 days. The results of the RP-HPLC and the nature of optional additives are shown in Table 4 below.

**Table 4.**

| Formation of Sulfoxide | | | | |
|---|---|---|---|---|
| Buffer % | pH | Additive | Sulfoxidated | |
| | | | 10d | 34d |
| Phosphate | 7.3 | - | 1.9 | 5.5 |
| Histidine | 7.3 | - | 0.9 | 2.4 |
| Histidine | 6.9 | - | 0.9 | 2.0 |
| Histidine | 6.5 | - | 0.8 | 1.9 |
| Histidine | 7.3 | 18 mM Met | 0.8 | 2.0 |
| Histidine | 7.3 | 18 mM Cys | 2.4 | 2.9 |
| Histidine | 7.3 | 0.42mM toc. | 1.1 | 3.0 |
| Histidine | 7.3 | 9% ethanol | 1.3 | 4.2 |
| Histidine | 7.3 | 18 mM asc. | 41 | nd |
| Histidine | 7.3 | 0.8% NaCl | 1.3 | 3.5 |
| Toc.: tocopherol; asc.: ascorbic acid. | | | | |

As compared with phosphate buffer, a marked reduction of the formation of sulfoxidated B-hGH is observed in histidine buffer (pH 7.3). A reduction of the formation of sulfoxide is observed with falling pH in His-buffer.
No further effect was obtained by addition of anti-oxidants or other additives.

### EXAMPLE 4

### Crystallization of hGH in the presence of phosphate or histidine buffer

Aliquots of a hGH solution prepared according to Dalboege et al Biotechnology (1987), 5, 161-164, in concentrations of 6 mg/ml were incubated in 10 mM phosphate or 10 mM histidine buffer at pH 6.2. To each of the samples was added ethanol to a final concentration of 7.5% (v/v), followed by addition of zinc acetate solution to a final zinc concentration of 1.34 mol Zn/mol hGH in the case of phosphate buffer and 5.5 mol Zn/mol hGH in the case of histidine buffer.

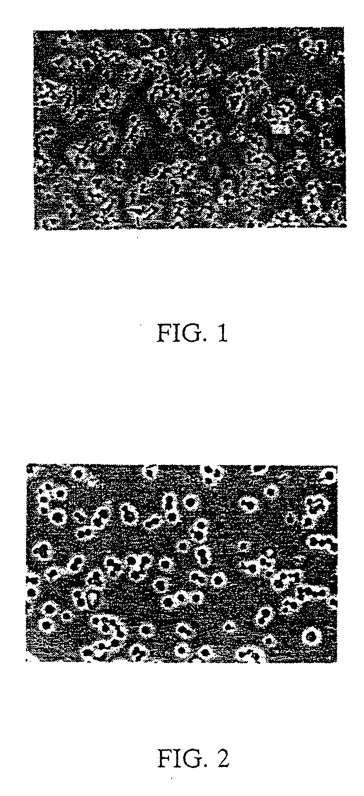

The crystals were grown in suspension for 16 hrs and the crystallization was monitored by phase contrast microscopy. The crystals formed in histidine buffer have a well defined unisized hexagonal appearance comprising little or no amorphous contaminants (Fig. 1). In contrast, hGH crystals formed in phosphate buffer under the exact identical conditions show a much more pronounced heterogeneous appearance comprising a considerable amount of amorphous material (Fig. 2).

The crystals were allowed to grow for a further 5 days. Crystals formed in both histidine and phosphate buffer were collected by centrifugation and the crystals were dissolved in 7 M urea followed by hGH analysis.

| Buffer | % crystals | % free hGH |
|---|---|---|
| Histidine | 65 | 35 |
| Phosphate | 55 | 45 |

Thus histidine buffer provides better conditions for hGH crystallization with respect to both yield of crystals and quality.

### EXAMPLE 5

### The stability of lyophilized hGH preparations comprising histidine and succcrose or mannitol compared to a conventional hGH preparation containing phosphate, glycine and mannitol.

The following preparations 1-8 were made by desalting a hGH solution into the stated histidine buffers. After adjusting the hGH concentration to 6 IU/ml with the various histidine buffers, the stated amounts of mannitol and sucrose were dissolved.

Preparation 9 corresponds to a conventional hGH preparation and is used as reference.

All hGH solutions 1-9 were filled into vials of 1 ml and lyophilized.

Analyses of hGH were performed after reconstitution with a 0.9% solution of benzyl alcohol.
1. hGH 6 IU/ml
   Adjusted to pH 6.5 using HCl
   Mannitol 33 mg/ml
2. hGH 6 IU/ml
   Adjusted to pH 6.5 using HCl
   Sucrose 62 mg/ml
3. hGH 6 IU/ml
   Adjusted to pH 7.0 using HCl
   Mannitol 33 mg/ml
4. hGH 6 IU/ml
   Adjusted to pH 7.0 using HCl
   Sucrose 62 mg/ml
5. hGH 6 IU/ml
   Adjusted to pH 6.5 using HCl
   Mannitol 33 mg/ml
6. hGH 6 IU/ml
   Adjusted to pH 6.5 using HCl
   Sucrose 62 mg/ml
7. hGH 6 IU/ml
   Adjusted to pH 7.0 using HCl
   Mannitol 33 mg/ml
8. hGH 6 IU/ml
   Adjusted to pH 7.0 using HCl
   Sucrose 62 mg/ml
9. hGH 6 IU7ml
   Na₂HPO₄, 2H₂O 0.59 mg/ml
   NaH₂PO₄, 2H₂O 0.53 mg/ml
   Mannitol, 20.5 mg/ml
   Adjusted to pH 7.0 using phosphoric acid

The lyophilized products are readily soluble and form clear aqueous solutions.

The amount of polymer before lyophilization (BL) and immediately after lyophilization, after 7 months at 4°C, after 7 months at 4°C plus 4 months at 37°C, and after 7 months at 4°C plus 4 months at 25°C in % is stated in Table 5.

The amount of dimer in % is stated in Table 6.
The amount of desamido hGH in % is stated in Table 7, and
The amount of sulfoxide in % is stated in Table 8.
The amount of desamido hGH and sulfoxide was determined as in Examples 1-4.

The amount of dimer and polymer was determined by gp-HPLC.

**Table 5**

| Buffer | Amount of Polymer in % | | | | |
|---|---|---|---|---|---|
| | BL | T=0 | 7 mos. 4°C | 7 mos. 4°C + 4 mos. 37°C | 7 mos. 4°C + 4 mos. 25°C |
| No. 1 | 0.2 | 1.5 1.5 | 2.0 | 6.0 | - |
| No. 2 | - | 0.2 0.2 | 0.2 | 0.2 | 0.2 |
| No. 3 | 0.2 | 0.8 1.0 | 1.8 | 4.0 | 2.6 |
| No. 4 | - | 0.2 <0.2 | 0.2 | <0.2 | <0.2 |
| No. 5 | <0.2 | 0.8 0.7 | 1.6 | 3.1 | 3.0 |
| No. 6 | - | 0.2 0.2 | 0.2 | 0.2 | <0.2 |
| No. 7 | 0.2 | 1.3 1.4 | 2.0 | 3.1 | 2.3 |
| No. 8 | - | 0.2 0.2 | 0.2 | <0.2 | <0.2 |
| No. 9 | 0.2 | 1.2 1.4 | 2.3 | 2.9 | 2.2 |

The amount of polymer is clearly lower for samples comprising sucrose.

**Table 6**

| Buffer | Amount of Dimer in % | | | | |
|---|---|---|---|---|---|
| | BL | T=0 | 7 mos. 4°C | 7 mos. 4°C + 4 mos. 37°C | 7 mos. 4°C + 4 mos. 25°C |
| No. 1 | 0.4 | 0.8 0.9 | 1.8 | 3.6 | - |
| No. 2 | - | 0.4 0.4 | 0.3 | 2.8 | 0.4 |
| No. 3 | 0.4 | 1.1 1.1 | 2.2 | 5.6 | 4.7 |
| No. 4 | - | 0.4 0.4 | 0.4 | 3.3 | 0.4 |
| No. 5 | 0.3 | 0.6 0.6 | 1.1 | 3.4 | 2.2 |
| No. 6 | - | 0.4 0.4 | 0.3 | 3.1 | 0.3 |
| No. 7 | 0.3 | 0.9 0.9 | 1.4 | 4.7 | 3.3 |
| No. 8 | - | 0.4 0.5 | 0.4 | 3.0 | 0.4 |
| No. 9 | 0.5 | 0.6 0.7 | 1.0 | 4.3 | 2.2 |

The amount of dimer is clearly lower for samples comprising sucrose.

**TABLE 7**

| Buffer | Amount of Desamido hGH in % | | | | |
|---|---|---|---|---|---|
| | BL | T=0 | 7 mos. 4°C | 7 mos. 4°C + 4 mos. 37°C | 7 mos. 4°C + 4 mos. 25°C |
| No. 1 | 2.0 | 1.8 1.4 | 1.1 | 3.9 | - |
| No. 2 | - | 1.4 1.5 | 3.1 | 22.8 | 1.3 |
| No. 3 | 2.1 | 1.2 1.3 | 2.1 | 5.6 | 1.5 |
| No. 4 | - | 1.6 1.6 | 2.1 | 23.6 | 1.0 |
| No. 5 | 1.6 | 1.3 0.9 | 1.4 | 12.1 | 1.0 |
| No. 6 | - | 1.2 1.3 | 1.6 | 23.0 | 1.4 |
| No. 7 | 2.0 | 1.4 1.6 | 1.0 | 4.8 | 4.6 |
| No. 8 | - | 1.5 1.4 | 1.9 | 20.2 | 2.9 |
| No. 9 | 2.1 | 1.7 1.5 | 2.0 | 9.9 | 3.6 |

The amount of desamido-hGH is very low for compositions comprising histidine after 7 months at 4°C + 4 months at 25°C.

**TABLE 8**

| Buffer | Amount of Sulfoxide in % | | | | |
|---|---|---|---|---|---|
| | BL | T=0 | 7 mos. 4°C | 7 mos. 4°C + 4 mos. 37°C | 7 mos. 4°C + 4 mos. 25°C |
| No. 1 | | | | | |
| No. 2 | | | | | 1.0 |
| No. 3 | | | | | 4.8 |
| No. 4 | | | | | 1.1 |
| No. 5 | | | | | |
| No. 6 | | | | | 1.0 |
| No. 7 | | | | | 1.8 |
| No. 8 | | | | | 1.4 |
| No. 9 | | | | | 2.4 |

The amount of sulfoxide is clearly lower in samples comprising sucrose.

### EXAMPLE 6

### Stability of lyophilized preparations comprising histidine, mannitol and disaccharide.

The following preparations were made in the same manner as disclosed in Example 5.
10. hGH 6 IU/ml
   Adjusted to pH 6.5 using HCl
   Sucrose 21 mg/ml
   Mannitol 22 mg/ml
11. hGH 6 IU/ml
   Adjusted to pH 7.0 using HCl
   Sucrose 21 mg/ml
   Mannitol 22 mg/ml
12. hGH 6 IU/ml
   Adjusted to pH 7.0 using HCl
   Trehalose 20 mg/ml
   Mannitol 22 mg/ml

The amount of desamido-hGH, polymer, and dimer in % was determined before lyophilization (BL), at t=0, after three months at 40°C and after 6 months at 25°C.

The results are shown in Tables 9-11 below.

It appears that samples comprising mannitol and sucrose or trehalose show better stability than samples comprising only mannitol as bulking agent for the lyophilization.

**Table 9**

| Buffer | Amount of Desamido-hGH in % | | | |
|---|---|---|---|---|
| | BL | T=0 | 3 mos. at 40°C | 6 mos. at 25°C |
| No. 1 | | 0.8 | 3.0 | 3.1 |
| No. 10 | 1.3 | 0.9 | 1.7 | 2.4 |
| No. 3 | | 0.9 | 2.4 | 3.0 |
| No. 11 | 0.9 | 1.4 | 1.2 | 2.0 |
| No. 12 | | 1.0 | 1.8 | 1.9 |
| No. 9 | 0.7 | 0.7 | 2.8 | 3.4 |

**Table 10**

| Buffer | Amount of Polymer in % | | | |
|---|---|---|---|---|
| | BL | T=0 | 3 mos. at 40°C | 6 mos. at 25°C |
| No. 1 | | 1.1 | 5.0 | 4.1 |
| No. 10 | 0.4 | 0.6 | 1.8 | 1.3 |
| No. 3 | | 0.9 | 5.2 | 3.2 |
| No. 11 | 0.5 | 0.6 | 1.5 | 1.1 |
| No. 12 | | 0.8 | 1.3 | 1.1 |
| No. 9 | 0.4 | 0.6 | 1.9 | 1.6 |

**Table 11**

| Buffer | Amount of Dimer in % | | | |
|---|---|---|---|---|
| | BL | T=0 | 3 mos. at 40°C | 6 mos. at 25°C |
| No. 1 | | 1.3 | 4.3 | 3.8 |
| No. 10 | 0.5 | 0.9 | 1.8 | 1.6 |
| No. 3 | | 1.3 | 5.0 | 4.3 |
| No. 11 | 0.6 | 1.2 | 2.2 | 1.8 |
| No. 12 | | 1.1 | 1.6 | 1.4 |
| No. 9 | 0.6 | 0.9 | 1.0 | 2.3 |

### EXAMPLE 7

### Formulation of a Pharmaceutical Preparation Containing Crystals of hGH:

Crystals were grown as described in example 4 and stored at 4°C. The crystals were then isolated by centrifugation and subsequent removal of the mother liquor. Then the crystals were lyophilized overnight to obtain dry crystals without remaining organic solvent. A pharmaceutical suspension of the dried crystals was prepared according to the following formulation:

| | |
|---|---|
| hGH crystals | 1.3 mg/ml |
| Histidine | 1.6 mg/ml |
| Zn(Ac)₂, H₂O | 0.1 mg/ml |
| Benzyl alcohol | 0.9% (v/v) |

pH was adjusted to 6.5 using HCl.

### EXAMPLE 8

Example 7 was repeated with the exception that Zn(Ac)₂, H₂O was omitted, giving a suspension of the following formulation:

| | |
|---|---|
| hGH crystals | 1.3 mg/ml |
| Histidine | 1.6 mg/ml |
| Benzyl alcohol | 0.9% (v/v) |

pH was adjusted to 6.2.

### EXAMPLE 9

The crystals were treated in the same way as in example 7 and the following suspension was formulated:

| | |
|---|---|
| hGH crystals | 1.3 mg/ml |
| Histidine | 1.33 mg/ml |
| NaCl | 5.7 mg/ml |
| Benzyl alcohol | 0.9% (v/v) |

pH was adjusted to 6.2.

### EXAMPLE 10

The crystals were treated in the same way as in example 7 and the following solution was prepared:

| | |
|---|---|
| hGH crystals | 1.3 mg/ml |
| Histidine | 1.14 mg/ml |
| NaCl | 9.0 mg/ml |

pH was adjusted to 6.1.

### EXAMPLE 11

In an analogous manner as described in EXAMPLE 1, biosynthetic human growth hormone was formulated in a concentration of 6 IU/ml in 0.9% benzyl alkohol at pH 6.5 in various concentrations of histidine: 0, 1, 2, 5, 10, 20, 30, 50, or 100 mM.

The samples were stored for 7 days at 37°C and analyses for the content of desamido, oxidized forms and dimers and polymers were performed in the same manner as described above. The results are shown in Table 12 below wherein the contents of desamido-hGH, dimers and polymers, and oxidized forms are determined by IE-HPLC, GP-HPLC and RP-HPLC and the contents of the cleaved forms of hGH is measured by IE-HPLC.

The amount of dimer is low when the concentration is 1 mM histidine or above. For formation of desamido compounds, concentrations of histidine of up to 30 mM give acceptable resuits, and for formation of oxidized forms, concentrations of histidine below 20 mM are preferred. An overall optimum is seen for a concentration of histidine of 5 mM.

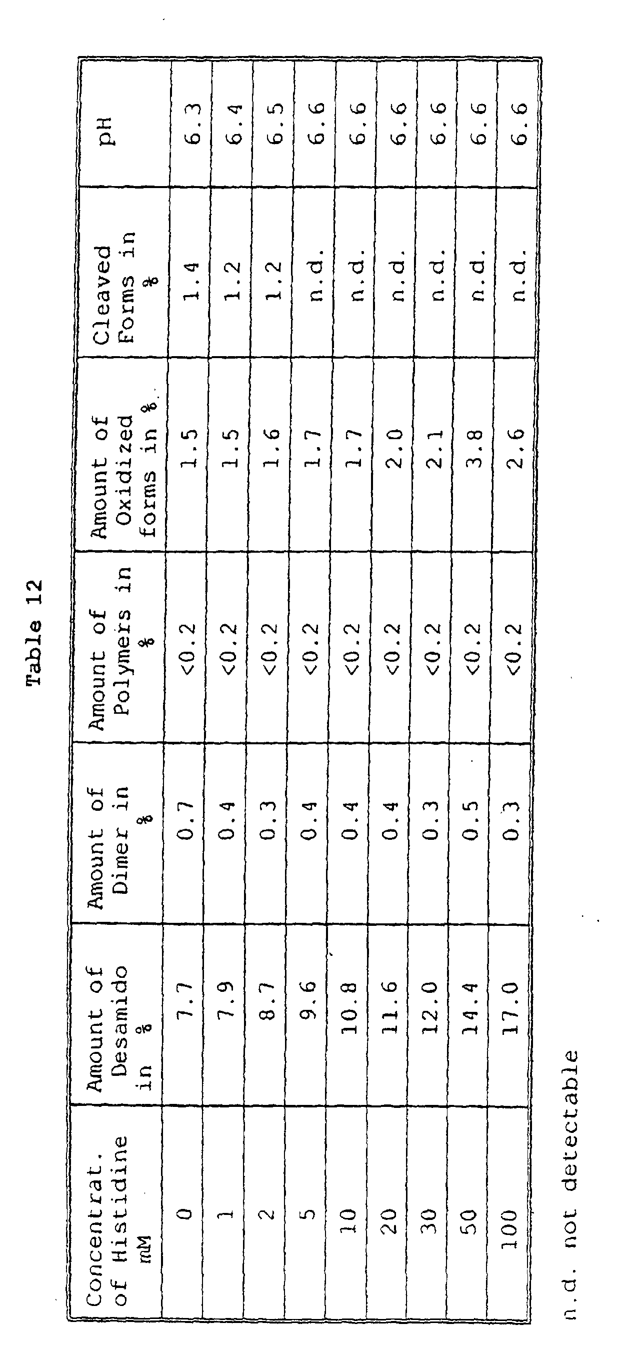

### REFERENCES

1) Y.-C.J. Wang and M.A. Hanson. Parenteral Formulations of Proteins and Peptides: Stability and Stabilizers. J. Parenteral Science and Technology 42 (Suppl.) (1988) 53-525.
2) M.C. Manning, K. Patel, R.T. Borchardt. Stability of Protein Pharmaceuticals. Pharmaceutical Research 6 (11) (1989) 903-918.
3) B.A. Johnson, J.M. Shirokawa, W.S. Hancock, M.W. Spellman, L.J. Basa and D.W. Asward. J.Biol.Chem. 264, 1462-71 (1989).
4) L.C. Teh et al., J.Biol.Chem., 262, 785-794 (1987).
5) G.W. Becker et al., Biotech.Appl.Biochem., 10, 326-337 (1988).
6) R.A. Houghten et al., Arch.Biochem.Biophys., 178, 350-355 (1977).
7) R.M. Riggin et al., Anal.Biochem., 167, 199-209 (1987).
8) P. Gellerfors et al., Acta Paediatr.Scand (suppl), 370, 93-100 (1990).
9) M.J. Kaufman, Pharm.Res., 7 (3) 289-292 (1990).

## Claims

1. A pharmaceutical preparation comprising human growth hormone and histidine or a derivative of histidine selected from the group of amides and esters of histidine such as the methyl or ethyl ester, dipeptides such as His-Gly, His-Ala, His-Leu, His-Lys, His-Ser and His-Phe, and analogues or derivatives of His such as imidazole, des-amino-His or poly-His, histidinol, imidazole-4-acetic acid and histamine, in an amount of from 0.1 to 12 mg histidine or derivative thereof per mg of growth hormone.

2. A pharmaceutical preparation according to claim 1 comprising histidine.

3. A pharmaceutical preparation in the form of a buffered aqueous solution of human growth hormone buffered with histidine buffer or a derivative of histidine selected from the group of amides and esters of histidine such as the methyl or ethyl ester, dipeptides such as His-Gly, His-Ala, His-Leu, His-Lys, His-Ser and His-Phe, and analogues or derivatives of His such as imidazole, des-amino-His or poly-His, histidinol, imidazole-4-acetic acid and histamine in a concentration from 1 mM to 100 mM, comprising growth hormone and histidine or derivative of histidine in an amount of from 0.1 to 12 mg histidine or derivative thereof per mg of growth hormone.

4. A pharmaceutical preparation according to claim 3 comprising histidine.

5. A pharmaceutical preparation according to claim 3 or 4, wherein the histidine or derivative of histidine is present in a concentration of from 2 to 20 mM.

6. A pharmaceutical preparation in the form of a lyophilised powder or "cake" comprising human growth hormone and histidine or a derivative of histidine selected from the group of amides and esters of histidine such as the methyl or ethyl ester, dipeptides such as His-Gly, His-Ala, His-Leu, His-Lys, His-Ser and His-Phe, and analogues or derivatives of His such as imidazole, des-amino-His or poly-His, histidinol, imidazole-4-acetic acid and histamine, in an amount from 0.1 to 12 mg histidine or derivative thereof per mg growth hormone, and a bulking agent for lyophilisation selected from the group consisting of sugar alcohols and disaccharides and mixtures thereof.

7. A pharmaceutical preparation according to claim 6, wherein the bulking agent is selected from the group consisting of mannitol and disaccharides and mixtures thereof.

8. A pharmaceutical preparation according to claim 7, wherein the disaccharide is sucrose or trehalose.

9. A pharmaceutical preparation in the form of a lyophilised powder to be reconstituted later using conventional vehicles such as distilled water or water for injection, comprising human growth hormone and histidine or a derivative of histidine selected from the group of amides and esters of histidine such as the methyl or ethyl ester, dipeptides such as His-Gly, His-Ala, His-Leu, His-Lys, His-Ser and His-Phe, and analogues or derivatives of His such as imidazole, des-amino-His or poly-His, histidinol, imidazole-4-acetic acid and histamine, in an amount of from 0.1 to 12 mg histidine or derivative thereof per mg of growth hormone.

10. A pharmaceutical preparation in the form of dried crystals of human growth hormone comprising histidine or a derivative of histidine selected from the group of amides and esters of histidine such as the methyl or ethyl ester, dipeptides such as His-Gly, His-Ala, His-Leu, His-Lys, His-Ser and His-Phe, and analogues or derivatives of His such as imidazole, des-amino-His or poly-His, histidinol, imidazole-4-acetic acid and histamine, in an amount of from 0.1 to 12 mg histidine or derivative thereof per mg of growth hormone.

11. A pharmaceutical preparation in the form of a buffered aqueous suspension of crystals of human growth hormone buffered with histidine buffer or a derivative of histidine selected from the group of amides and esters of histidine such as the methyl or ethyl ester, dipeptides such as His-Gly, His-Ala, His-Leu, His-Lys, His-Ser and His-Phe, and analogues or derivatives of His such as imidazole, des-amino-His or poly-His, histidinol, imidazole-4-acetic acid and histamine, comprising growth hormone and histidine or a derivative of histidine in an amount of from 0.1 to 12 mg histidine or derivative thereof per mg of growth hormone.

12. A preparation according to any of claims 8-10 comprising histidine.

13. A pharmaceutical preparation according to any of claims 3, 5-9 and 11, wherein the derivative of histidine is selected from the group of amides and esters of histidine such as the methyl or ethyl ester, dipeptides such as His-Gly, His-Ala, His-Leu, His-Lys, His-Ser and His-Phe, and analogues or derivatives of His such as imidazole, des-amino-His or poly-His.

14. A pharmaceutical preparation according to any of claims 3, 5-9 and 11, wherein the derivative of histidine is selected from the group of His-Gly, His-Ala, His-Leu, His-Lys, His-Phe, His-Ser, histidine methyl ester, histidinol, imidazole, imidazole-4-acetic acid and histamine.

15. A pharmaceutical preparation according to any of claims 1-14, wherein the pH is adjusted to a value in the interval 2-9.

16. A pharmaceutical preparation according to claim 15, wherein the pH is adjusted to a value in the interval from 5 to 8.

17. A pharmaceutical preparation according to any of claims 1-5 or 9-16, further comprising a sugar alcohol or a disaccharide or a mixture thereof.

18. A pharmaceutical preparation according to claim 17, comprising mannitol or a disaccharide or a mixture thereof.

19. A pharmaceutical preparation according to claim 18, wherein the disaccharide is sucrose or trehalose.

20. Crystals of human growth hormone comprising histidine or a derivative of histidine selected from the group of amides and esters of histidine such as the methyl or ethyl ester, dipeptides such as His-Gly, His-Ala, His-Leu, His-Lys, His-Ser and His-Phe, and analogues or derivatives of His such as imidazole, des-amino-His or poly-His, histidinol, imidazole-4-acetic acid and histamine, in an amount of from 0.1 to 12 mg histidine or derivative thereof per mg of growth hormone.

21. Crystals of human growth hormone and histidine or a derivative of histidine selected from the group of amides and esters of histidine such as the methyl or ethyl ester, dipeptides such as His-Gly, His-Ala, His-Leu, His-Lys, His-Ser and His-Phe, and analogues or derivatives of His such as imidazole, des-amino-His or poly-His, histidinol, imidazole-4-acetic acid and histamine, in an amount of from 0.1 to 12 mg histidine or derivative thereof per mg of growth hormone, prepared by a method comprising the steps of:
a) forming a solution of growth hormone in a solvent and adding histidine or a derivative of histidine and optionally adjusting the pH to a value from 5 to 8 using hydrochloric acid,
b) adding organic or inorganic cations,
c) crystallising the solution at a temperature from about 0°C to about 30°C, and
d) isolating the crystals formed by a manner known per se.

22. Crystals according to claim 20 or 21 comprising histidine.

23. A method of preparing crystals of human growth hormone and histidine or a derivative of histidine selected from the group of amides and esters of histidine such as the methyl or ethyl ester, dipeptides such as His-Gly, His-Ala, His-Leu, His-Lys, His-Ser and His-Phe, and analogues or derivatives of His such as imidazole, des-amino-His or poly-His, histidinol, imidazole-4-acetic acid and histamine, in an amount of from 0.1 to 12 mg histidine or derivative thereof per mg of growth hormone, comprising the steps of:
a) forming a solution of growth hormone in a solvent and adding histidine or a derivative of histidine and optionally adjusting the pH to a value from 5 to 8 using hydrochloric acid,
b) adding organic or inorganic cations,
c) crystallising the solution at a temperature from about 0°C to about 30°C, and
d) isolating the crystals formed by a manner known per se.

24. A method according to claim 23, wherein the solvent in step a) is selected from short chained aliphatic, alicyclic and aromatic alcohols and ketones.

25. A method according to claim 23 or 24, comprising addition of histidine in step a).

## Patentansprüche

1. Pharmazeutische Zubereitung umfassend menschliches Wachstumshormon und Histidin oder ein Derivat von Histidin, ausgewählt aus der Gruppe von Amiden und Estern von Histidin wie dem Methyl- oder Ethylester, Dipeptiden wie His-Gly, His-Ala, His-Leu, His-Lys, His-Ser und His-Phe und Analoga oder Derivaten von His wie Imidazol, Desamino-His oder Poly-His, Histidinol, Imidazol-4-essigsäure und Histamin in einer Menge von 0,1 bis 12 mg Histidin oder eines Derivates hiervon pro mg Wachstumshormon.

2. Pharmazeutische Zubereitung gemäß Anspruch 1 umfassend Histidin.

3. Pharmazeutische Zubereitung in Form einer gepufferten wässrigen Lösung von menschlichem Wachstumshormon, gepuffert mit Histidinpuffer oder einem Derivat von Histidin, ausgewählt aus der Gruppe von Amiden und Estern von Histidin wie dem Methyl- oder Ethylester, Dipeptiden wie His-Gly, His-Ala, His-Leu, His-Lys, His-Ser und His-Phe und Analoga oder Derivaten von His wie Imidazol, Desamino-His oder Poly-His, Histidinol, Imidazol-4-essigsäure und Histamin in einer Konzentration von 1 mM bis 100 mM, umfassend Wachstumshormon und Histidin oder ein Derivat von Histidin in einer Menge von 0,1 bis 12 mg Histidin oder eines Derivates hiervon pro mg Wachstumshormon.

4. Pharmazeutische Zubereitung gemäß Anspruch 3 umfassend Histidin.

5. Pharmazeutische Zubereitung gemäß Anspruch 3 oder 4, wobei Histidin oder ein Derivat von Histidin in einer Konzentration von 2 bis 20 mM vorliegt.

6. Pharmazeutische Zubereitung in Form eines lyophilisierten Pulvers oder "Kuchens" umfassend menschliches Wachstumshormon und Histidin oder ein Derivat von Histidin, ausgewählt aus der Gruppe von Amiden und Estern von Histidin wie dem Methyl- oder Ethylester, Dipeptiden wie His-Gly, His-Ala, His-Leu, His-Lys, His-Ser und His-Phe und Analoga oder Derivaten von His wie Imidazol, Desamino-His oder Poly-His, Histidinol, Imidazol-4-essigsäure und Histamin in einer Menge von 0,1 bis 12 mg Histidin oder eines Derivates hiervon pro mg Wachstumshormon und einen Füllstoff zur Lyophilisierung, ausgewählt aus der Gruppe bestehend aus Zuckeralkoholen und Disacchariden und Mischungen hiervon.

7. Pharmazeutische Zubereitung gemäß Anspruch 6, wobei der Füllstoff ausgewählt ist aus der Gruppe bestehend aus Mannitol und Disacchariden und Mischungen hiervon.

8. Pharmazeutische Zubereitung gemäß Anspruch 7, wobei das Disaccharid Saccharose oder Trehalose ist.

9. Pharmazeutische Zubereitung in Form eines lyophilisierten Pulvers, das später unter Verwendung konventioneller Vehikel wie destilliertem Wasser oder Wasser zur Injektion rekonstituiert werden soll, umfassend menschliches Wachstumshormon und Histidin oder ein Derivat von Histidin, ausgewählt aus der Gruppe von Amiden und Estern von Histidin wie dem Methyl- oder Ethylester, Dipeptiden wie His-Gly, His-Ala, His-Leu, His-Lys, His-Ser und His-Phe und Analoga oder Derivaten von His wie Imidazol, Desamino-His oder Poly-His, Histidinol, Imidazol-4-essigsäure und Histamin in einer Menge von 0,1 bis 12 mg Histidin oder eines Derivates hiervon pro mg Wachstumshormon.

10. Pharmazeutische Zubereitung in Form von getrockneten Kristallen von menschlichem Wachstumshormon umfassend Histidin oder ein Derivat von Histidin, ausgewählt aus der Gruppe von Amiden und Estern von Histidin wie dem Methyl- oder Ethylester, Dipeptiden wie His-Gly, His-Ala, His-Leu, His-Lys, His-Ser und His-Phe und Analoga oder Derivaten von His wie Imidazol, Desamino-His oder Poly-His, Histidinol, Imidazol-4-essigsäure und Histamin in einer Menge von 0,1 bis 12 mg Histidin oder eines Derivates hiervon pro mg Wachstumshormon.

11. Pharmazeutische Zubereitung in Form einer gepufferten wässrigen Suspension von Kristallen von menschlichem Wachstumshormon, gepuffert mit Histidinpuffer oder einem Derivat von Histidin, ausgewählt aus der Gruppe von Amiden und Estern von Histidin wie dem Methyl- oder Ethylester, Dipeptiden wie His-Gly, His-Ala, His-Leu, His-Lys, His-Ser und His-Phe und Analoga oder Derivaten von His wie Imidazol, Desamino-His oder Poly-His, Histidinol, Imidazol-4-essigsäure und Histamin, umfassend Wachstumshormon und Histidin oder ein Derivat von Histidin in einer Menge von 0,1 bis 12 mg Histidin oder eines Derivates hiervon pro mg Wachstumshormon.

12. Zubereitung gemäß einem der Ansprüche 8-10 umfassend Histidin.

13. Pharmazeutische Zubereitung gemäß einem der Ansprüche 3, 5-9 und 11, wobei das Derivat von Histidin ausgewählt ist aus der Gruppe von Amiden und Estern von Histidin wie dem Methyl- oder Ethylester, Dipeptiden wie His-Gly, His-Ala, His-Leu, His-Lys, His-Ser und His-Phe und Analoga oder Derivaten von His wie Imidazol, Desamino-His oder Poly-His.

14. Pharmazeutische Zubereitung gemäß einem der Ansprüche 3, 5-9 und 11, wobei das Derivat von Histidin ausgewählt ist aus der Gruppe von His-Gly, His-Ala, His-Leu, His-Lys, His-Phe, His-Ser, Histidinmethylester, Histidinol, Imidazol, Imidazol-4-essigsäure und Histamin.

15. Pharmazeutische Zubereitung gemäß einem der Ansprüche 1-14, wobei der pH auf einen Wert im Intervall 2-9 eingestellt ist.

16. Pharmazeutische Zubereitung gemäß Anspruch 15, wobei der pH auf einen Wert im Intervall von 5 bis 8 eingestellt ist.

17. Pharmazeutische Zubereitung gemäß einem der Ansprüche 1-5 oder 9-16, weiterhin umfassend einen Zuckeralkohol oder ein Disaccharid oder eine Mischung hiervon.

18. Pharmazeutische Zubereitung gemäß Anspruch 17, umfassend Mannitol oder ein Disaccharid oder eine Mischung hiervon.

19. Pharmazeutische Zubereitung gemäß Anspruch 18, wobei das Disaccharid Saccharose oder Trehalose ist.

20. Kristalle von menschlichem Wachstumshormon umfassend Histidin oder ein Derivat von Histidin ausgewählt aus der Gruppe von Amiden und Estern von Histidin wie dem Methyl- oder Ethylester, Dipeptiden wie His-Gly, His-Ala, His-Leu, His-Lys, His-Ser und His-Phe und Analoga oder Derivaten von His wie Imidazol, Desamino-His oder Poly-His, Histidinol, Imidazol-4-essigsäure und Histamin in einer Menge von 0,1 bis 12 mg Histidin oder eines Derivates hiervon pro mg Wachstumshormon.

21. Kristalle von menschlichem Wachstumshormon und Histidin oder einem Derivat von Histidin ausgewählt aus der Gruppe von Amiden und Estern von Histidin wie dem Methyl- oder Ethylester, Dipeptiden wie His-Gly, His-Ala, His-Leu, His-Lys, His-Ser und His-Phe und Analoga oder Derivaten von His wie Imidazol, Desamino-His oder Poly-His, Histidinol, Imidazol-4-essigsäure und Histamin in einer Menge von 0,1 bis 12 mg Histidin oder eines Derivates hiervon pro mg Wachstumshormon, hergestellt durch ein Verfahren umfassend die Schritte:
a) Bilden einer Lösung von Wachstumshormon in einem Lösungsmittel und Zufügen von Histidin oder einem Derivat von Histidin und wahlweise Einstellen des pH auf einen Wert von 5 bis 8 unter Verwendung von Salzsäure,
b) Zufügen organischer oder anorganischer Kationen,
c) Kristallisieren der Lösung bei einer Temperatur von ungefähr 0°C bis ungefähr 30 °C und
d) Isolieren der gebildeten Kristalle auf eine an sich bekannte Weise.

22. Kristalle gemäß Anspruch 20 oder 21 umfassend Histidin.

23. Verfahren zur Herstellung von Kristallen von menschlichem Wachstumshormon und Histidin oder einem Derivat von Histidin ausgewählt aus der Gruppe von Amiden und Estern von Histidin wie dem Methyl- oder Ethylester, Dipeptiden wie His-Gly, His-Ala, His-Leu, His-Lys, His-Ser und His-Phe und Analoga oder Derivaten von His wie Imidazol, Desamino-His oder Poly-His, Histidinol, Imidazol-4-essigsäure und Histamin in einer Menge von 0,1 bis 12 mg Histidin oder eines Derivates hiervon pro mg Wachstumshormon, umfassend die Schritte:
a) Bilden einer Lösung von Wachstumshormon in einem Lösungsmittel und Zufügen von Histidin oder einem Derivat von Histidin und wahlweise Einstellen des pH auf einen Wert von 5 bis 8 unter Verwendung von Salzsäure,
b) Zufügen organischer oder anorganischer Kationen,
c) Kristallisieren der Lösung bei einer Temperatur von ungefähr 0 °C bis ungefähr 30 °C und
d) Isolieren der gebildeten Kristalle auf eine an sich bekannte Weise.

24. Verfahren gemäß Anspruch 23, wobei das Lösungsmittel in Schritt a) ausgewählt ist aus kurzkettigen aliphatischen, alizyklischen und aromatischen Alkoholen und Ketonen.

25. Verfahren gemäß Anspruch 23 oder 24 umfassend das Zufügen von Histidin in Schritt a).

## Revendications

1. Une préparation pharmaceutique comprenant de l'hormone de croissance humaine et de l'histidine ou un dérivé d'histidine choisi parmi le groupe des amides et des esters d'histidine comme l'ester de méthyle ou d'éthyle, des dipeptides comme His-Gly, His-Ala, His-Leu, His-Lys, His-Ser et His-Phe et les analogues ou dérivés de His comme l'imidazole, le des-amino-His ou le poly-His, l'histidinol, l'acide imidazole-4-acétique et l'histamine selon une quantité de 0,1 à 12 mg d'histidine ou d'un dérivé de celle-ci par mg d'hormone de croissance.

2. Une préparation pharmaceutique selon la revendication 1 comprenant de l'histidine.

3. Une préparation pharmaceutique sous la forme d'une solution aqueuse tamponnée d'hormone de croissance humaine tamponnée avec un tampon d'histidine ou un dérivé d'histidine choisi parmi le groupe des amides et des esters d'histidine comme l'ester de méthyle ou d'éthyle, les dipeptides comme His-Gly, His-Ala, His-Leu, His-Lys, His-Ser et His-Phe et les analogues ou dérivés de His comme l'imidazole, le des-amino-His ou le poly-His, l'histidinol, l'acide imidazole-4-acétique et l'histamine selon une concentration de 1 mM à 100 mM comprenant l'hormone de croissance et l'histidine ou un dérivé de l'histidine selon une quantité de 0,1 à 12 mg d'histidine ou d'un dérivé de celle-ci par mg d'hormone de croissance.

4. Une préparation pharmaceutique selon la revendication 3 comprenant de l'histidine.

5. Une préparation pharmaceutique selon la revendication 3 ou 4, dans laquelle l'histidine ou le dérivé d'histidine est présent selon une concentration de 2 à 20 mM.

6. Une préparation pharmaceutique sous la forme d'une poudre lyophilisée ou "gâteau" comprenant de l'hormone de croissance humaine et de l'histidine ou un dérivé d'histidine choisi parmi le groupe des amides et des esters d'histidine comme l'ester de méthyle ou d'éthyle, les dipeptides comme His-Gly, His-Ala, His-Leu, His-Lys, His-Ser et His-Phe et les analogues ou dérivés de His comme l'imidazole, le des-amino-His ou le poly-His, l'histidinol, l'acide imidazole-4-acétique et l'histamine selon une quantité de 0,1 à 12 mg d'histidine ou de son dérivé par mg d'hormone de croissance et un agent conférant de la masse pour la lyophilisation choisi parmi le groupe comprenant les alcools-sucre et les disaccharides et leurs mélanges.

7. Une préparation pharmaceutique selon la revendication 6, dans laquelle l'agent conférant de la masse est choisi parmi le groupe comprenant le mannitol et les disaccharides et leurs mélanges.

8. Une préparation pharmaceutique selon la revendication 7, dans laquelle le disaccharide est du saccharose ou du tréhalose.

9. Une préparation pharmaceutique sous la forme d'une poudre lyophilisée à reconstituer ultérieurement en utilisant des véhicules classiques comme de l'eau distillée ou de l'eau pour injection comprenant de l'hormone de croissance humaine et de l'histidine ou un dérivé d'histidine choisi parmi le groupe des amides et des esters d'histidine comme l'ester de méthyle ou d'éthyle, les dipeptides comme His-Gly, His-Ala, His-Leu, His-Lys, His-Ser et His-Phe et les analogues ou dérivés de His comme l'imidazole, le des-amino-His ou le poly-His, l'histidinol, l'acide imidazole-4-acétique et l'histamine selon une quantité de 0,1 à 12 mg d'histidine ou d'un dérivé de celle-ci par mg d'hormone de croissance.

10. Une préparation pharmaceutique sous la forme de cristaux séchés d'hormone de croissance humaine comprenant de l'histidine ou un dérivé d'histidine choisi parmi le groupe des amides et des esters d'histidine comme l'ester de méthyle ou d'éthyle, les dipeptides comme His-Gly, His-Ala, His-Leu, His-Lys, His-Ser et His-Phe et les analogues ou dérivés de His comme l'imidazole, le des-amino-His ou le poly-His, l'histidinol, l'acide imidazole-4-acétique et l'histamine, selon une quantité de 0,1 à 12 mg d'histidine ou d'un dérivé de celle-ci par mg d'hormone de croissance.

11. Une préparation pharmaceutique sous la forme d'une suspension aqueuse tamponnée de cristaux d'hormone de croissance humaine tamponnés avec un tampon d'histidine ou un dérivé d'histidine choisi parmi le groupe des amides et des esters d'histidine comme l'ester de méthyle ou d'éthyle, les dipeptides comme His-Gly, His-Ala, His-Leu, His-Lys, His-Ser et His-Phe et les analogues ou dérivés de His comme l'imidazole, le des-amino-His ou le poly-His, l'histidinol, l'acide imidazole-4-acétique et l'histamine, comprenant de l'hormone de croissance et de l'histidine ou un dérivé d'histidine selon une quantité de 0,1 à 12 mg d'histidine ou d'un dérivé de celle-ci par mg d'hormone de croissance.

12. Une préparation selon l'une quelconque des revendications 8 à 10 comprenant de l'histidine.

13. Une préparation pharmaceutique selon l'une quelconque des revendications 3, 5 à 9 et 11, dans laquelle le dérivé d'histidine est choisi parmi le groupe des amides et des esters de l'histidine comme l'ester de méthyle ou d'éthyle, les dipeptides comme His-Gly, His-Ala, His-Leu, His-Lys, His-Ser et His-Phe et les analogues ou dérivés de His comme l'imidazole, le des-amino-His ou le poly-His.

14. Une préparation pharmaceutique selon l'une quelconque des revendications 3, 5 à 9 et 11, dans laquelle le dérivé d'histidine est choisi parmi le groupe de His-Gly, His-Ala, His-Leu, His-Lys, His-Phe, His-Ser, l'ester de méthyle d'histidine, l'histidinol, l'imidazole, l'acide imidazole-4-acétique et l'histamine.

15. Une préparation pharmaceutique selon l'une quelconque des revendications 1 à 14, dans laquelle le pH est ajusté à une valeur comprise dans l'intervalle de 2-9.

16. Une préparation pharmaceutique selon la revendication 15, dans laquelle le pH est ajusté à une valeur comprise dans l'intervalle de 5 à 8.

17. Une préparation pharmaceutique selon l'une quelconque des revendications 1 à 5 ou 9 à 16, comprenant en outre un alcool-sucre ou un disaccharide ou un mélange de celui-ci.

18. Une préparation pharmaceutique selon la revendication 17, comprenant du mannitol ou un disaccharide ou un mélange de celui-ci.

19. Une préparation pharmaceutique selon la revendication 18, dans laquelle le disaccharide est du saccharose ou du tréhalose.

20. Cristaux d'hormone de croissance humaine comprenant de l'histidine ou un dérivé d'histidine choisi parmi le groupe des amides et des esters de l'histidine comme l'ester de méthyle-ou d'éthyle, les dipeptides comme His-Gly, His-Ala, His-Leu, His-Lys, His-Ser et His-Phe et les analogues ou dérivés de His comme l'imidazole, le des-amino-His ou le poly-His, l'histidinol, l'acide imidazole-4-acétique et l'histamine selon une quantité de 0,1 à 12 mg d'histidine ou d'un dérivé de celle-ci par mg d'hormone de croissance.

21. Cristaux d'hormone de croissance humaine et d'histidine ou d'un dérivé d'histidine choisi parmi le groupe des amides et des esters d'histidine comme l'ester de méthyle ou d'éthyle, les dipeptides comme His-Gly, His-Ala, His-Leu, His-Lys, His-Ser et His-Phe et les analogues ou dérivés de His comme l'imidazole, le des-amino-His ou le poly-His, l'histidinol, l'acide imidazole-4-acétique et l'histamine, selon une quantité de 0,1 à 12 mg d'histidine ou d'un dérivé de celle-ci par mg d'hormone de croissance, préparés par un procédé comprenant les étapes consistant à :
a) former une solution d'hormone de croissance dans un solvant et ajouter de l'histidine ou un dérivé d'histidine et éventuellement ajuster le pH à une valeur de 5 à 8 en utilisant de l'acide chlorhydrique,
b) ajouter des cations organiques ou non organiques,
c) cristalliser la solution à une température d'environ 0°C à environ 30°C et
d) isoler les cristaux formés d'une façon connue en soi.

22. Cristaux selon la revendication 20 ou 21 comprenant de l'histidine.

23. Un procédé de préparation de cristaux d'hormone de croissance humaine et d'histidine ou d'un dérivé d'histidine choisi parmi le groupe des amides et des esters de l'histidine comme l'ester de méthyle ou d'éthyle, les dipeptides comme His-Gly, His-Ala, His-Leu, His-Lys, His-Ser et His-Phe et les analogues ou dérivés de His comme l'imidazole, le des-amino-His ou le poly-His, l'histidinol, l'acide imidazole-4-acétique et l'histamine selon une quantité de 0,1 à 12 mg d'histidine ou d'un dérivé de celle-ci par mg d'hormone de croissance, comprenant les étapes consistant à :
a) former une solution d'hormone de croissance dans un solvant et ajouter de l'histidine ou un dérivé d'histidine et éventuellement ajuster le pH à une valeur de 5 à 8 en utilisant de l'acide chlorhydrique,
b) ajouter des cations organiques ou non organiques,
c) cristalliser la solution à une température d'environ 0°C à environ 30°C et
d) isoler les cristaux formés d'une façon connue en soi.

24. Un procédé selon la revendication 23, dans lequel le solvant dans l'étape a) est choisi parmi les alcools et les cétones aromatiques et alicycliques, aliphatiques à chaîne courte.

25. Un procédé selon la revendication 23 ou 24 comprenant l'addition d'histidine dans l'étape a).
